# EUROPEAN PATENT APPLICATION

(11) **EP 3 533 469 A1**
(43) Date of publication of application: **04.09.2019**
(21) Application number: 17863032.3
(22) Date of filing: 18.10.2017
(51) Int. Cl.: A61K 45/00, A61K 39/395, A61P 9/10, C12Q 1/68, G01N 33/53

(54) **ANTI-ATHEROSCLEROTIC AGENT AND SYMPTOM IDENTIFICATION METHOD FOR ARTERIOSCLEROSIS**

(30) Priority: 19.10.2016 JP 2016204783
(71) Applicant: Public University Corporation Yokohama City University, Yokohama-shi, Kanagawa 236-0027 (JP)
(72) Inventor: ISHIGAMI, Tomoaki, Yokohama-shi Kanagawa 236-0004 (JP); CHEN, Lin, Yokohama-shi Kanagawa 236-0004 (JP)
(74) Representative: Mewburn Ellis LLP
(86) International application number: PCT/JP2017/037624
(87) International publication number: WO 2018/074497

(57) **Abstract**

A novel means that can suppress development of arteriosclerotic lesions and treat arteriosclerosis is disclosed. An antiarteriosclerotic agent according to the present invention comprises, as an active ingredient, a substance which selectively suppresses B2 cells. The substance which selectively suppresses B2 cells is, for example, an anti-CD23 antibody. A first aspect of the method of assisting in determining a condition of arteriosclerosis according to the present invention comprises measuring expression of at least one gene selected from particular gene groups using a sample isolated from a subject. A second aspect of the method comprises measuring the level of IgG3 and/or the level of total IgG in a serum sample isolated from a subject.

SEQUENCE LISTING

<110>  Public University Corporation Yokohama City University

<120>  Anti-arteriosclerosis agent and method for determination of
       condition of arteriosclerosis

<130>  PF606-PCT

<160>  4

<170>  PatentIn version 3.5

<210>  1
<211>  22
<212>  DNA
<213>  Artificial Sequence

<220>
<223>  primer I-Aa forward

<400>  1
aaattccacc ccagctacca at                                                22

<210>  2
<211>  18
<212>  DNA
<213>  Artificial Sequence

<220>
<223>  primer I-Aa reverse

<400>  2
gctgacccag cagcacag                                                     18

<210>  3
<211>  1620
<212>  DNA
<213>  Homo sapiens

<220>
<221>  CDS
<222>  (214)..(1179)

<400>  3
agtggctcta ctttcagaag aaagtgtctc tcttcctgct taaacctctg tctctgacgg      60

tccctgccaa tcgctctggt cgaccccaac acactaggag gacagacaca ggctccaaac     120

tccactaacc agagctgtga ttgtgcccgc tgagtggact gcgttgtcag ggagtgagtg     180

ctccatcatc gggagaatcc aagcaggacc gcc atg gag gaa ggt caa tat tca      234
                                     Met Glu Glu Gly Gln Tyr Ser
                                     1               5

gag atc gag gag ctt ccc agg agg cgg tgt tgc agg cgt ggg act cag       282
Glu Ile Glu Glu Leu Pro Arg Arg Arg Cys Cys Arg Arg Gly Thr Gln
        10                  15                  20

atc gtg ctg ctg ggg ctg gtg acc gcc gct ctg tgg gct ggg ctg ctg       330

Ile Val Leu Leu Gly Leu Val Thr Ala Ala Leu Trp Ala Gly Leu Leu
    25                  30                  35

act ctg ctt ctc ctg tgg cac tgg gac acc aca cag agt cta aaa cag      378
Thr Leu Leu Leu Leu Trp His Trp Asp Thr Thr Gln Ser Leu Lys Gln
40                  45                  50                  55

ctg gaa gag agg gct gcc cgg aac gtc tct caa gtt tcc aag aac ttg      426
Leu Glu Glu Arg Ala Ala Arg Asn Val Ser Gln Val Ser Lys Asn Leu
                60                  65                  70

gaa agc cac cac ggt gac cag atg gcg cag aaa tcc cag tcc acg cag      474
Glu Ser His His Gly Asp Gln Met Ala Gln Lys Ser Gln Ser Thr Gln
            75                  80                  85

att tca cag gaa ctg gag gaa ctt cga gct gaa cag cag aga ttg aaa      522
Ile Ser Gln Glu Leu Glu Glu Leu Arg Ala Glu Gln Gln Arg Leu Lys
        90                  95                  100

tct cag gac ttg gag ctg tcc tgg aac ctg aac ggg ctt caa gca gat      570
Ser Gln Asp Leu Glu Leu Ser Trp Asn Leu Asn Gly Leu Gln Ala Asp
    105                 110                 115

ctg agc agc ttc aag tcc cag gaa ttg aac gag agg aac gaa gct tca      618
Leu Ser Ser Phe Lys Ser Gln Glu Leu Asn Glu Arg Asn Glu Ala Ser
120                 125                 130                 135

gat ttg ctg gaa aga ctc cgg gag gag gtg aca aag cta agg atg gag      666
Asp Leu Leu Glu Arg Leu Arg Glu Glu Val Thr Lys Leu Arg Met Glu
                140                 145                 150

ttg cag gtg tcc agc ggc ttt gtg tgc aac acg tgc cct gaa aag tgg      714
Leu Gln Val Ser Ser Gly Phe Val Cys Asn Thr Cys Pro Glu Lys Trp
            155                 160                 165

atc aat ttc caa cgg aag tgc tac tac ttc ggc aag ggc acc aag cag      762
Ile Asn Phe Gln Arg Lys Cys Tyr Tyr Phe Gly Lys Gly Thr Lys Gln
        170                 175                 180

tgg gtc cac gcc cgg tat gcc tgt gac gac atg gaa ggg cag ctg gtc      810
Trp Val His Ala Arg Tyr Ala Cys Asp Asp Met Glu Gly Gln Leu Val
    185                 190                 195

agc atc cac agc ccg gag gag cag gac ttc ctg acc aag cat gcc agc      858
Ser Ile His Ser Pro Glu Glu Gln Asp Phe Leu Thr Lys His Ala Ser
200                 205                 210                 215

cac acc ggc tcc tgg att ggc ctt cgg aac ttg gac ctg aag ggg gag      906
His Thr Gly Ser Trp Ile Gly Leu Arg Asn Leu Asp Leu Lys Gly Glu
                220                 225                 230

ttt atc tgg gtg gat ggg agc cac gtg gac tac agc aac tgg gct cca      954
Phe Ile Trp Val Asp Gly Ser His Val Asp Tyr Ser Asn Trp Ala Pro
            235                 240                 245

ggg gag ccc acc agc cgg agc cag ggc gag gac tgc gtg atg atg cgg     1002
Gly Glu Pro Thr Ser Arg Ser Gln Gly Glu Asp Cys Val Met Met Arg
        250                 255                 260

ggc tcc ggt cgc tgg aac gac gcc ttc tgc gac cgt aag ctg ggc gcc     1050
Gly Ser Gly Arg Trp Asn Asp Ala Phe Cys Asp Arg Lys Leu Gly Ala
    265                 270                 275

tgg gtg tgc gac cgg ctg gcc aca tgc acg ccg cca gcc agc gaa ggt     1098
Trp Val Cys Asp Arg Leu Ala Thr Cys Thr Pro Pro Ala Ser Glu Gly
280                 285                 290                 295

tcc gcg gag tcc atg gga cct gat tca aga cca gac cct gac ggc cgc     1146
Ser Ala Glu Ser Met Gly Pro Asp Ser Arg Pro Asp Pro Asp Gly Arg
                300                 305                 310

ctg ccc acc ccc tct gcc cct ctc cac tct tga gcatggatac agccaggccc     1199
Leu Pro Thr Pro Ser Ala Pro Leu His Ser
            315                 320

agagcaagac cctgaagacc cccaaccacg gcctaaaagc ctctttgtgg ctgaaaggtc     1259

cctgtgacat tttctgccac ccaaacggag gcagctgaca catctcccgc tcctctatgg     1319

cccctgcctt cccaggagta caccccaaca gcaccctctc cagatgggag tgcccccaac     1379

agcaccctct ccagatgaga gtacacccca acagcaccct ctccagatga gagtacaccc     1439

caacagcacc ctctccagat gagagtacac cccaacagca ccctctccag atgcagcccc     1499

atctcctcag caccccagga cctgagtatc cccagctcag gtggtgagtc ctcctgtcca     1559

gcctgcatca ataaaatggg gcagtgatgg cctcccacat ttgtcccctt cttggaaaaa     1619

a                                                                   1620

<210>  4
<211>  321
<212>  PRT
<213>  Homo sapiens

<400>  4

Met Glu Glu Gly Gln Tyr Ser Glu Ile Glu Glu Leu Pro Arg Arg Arg
1               5                   10                  15

Cys Cys Arg Arg Gly Thr Gln Ile Val Leu Leu Gly Leu Val Thr Ala
            20                  25                  30

Ala Leu Trp Ala Gly Leu Leu Thr Leu Leu Leu Leu Trp His Trp Asp
        35                  40                  45

Thr Thr Gln Ser Leu Lys Gln Leu Glu Glu Arg Ala Ala Arg Asn Val
    50                  55                  60

Ser Gln Val Ser Lys Asn Leu Glu Ser His His Gly Asp Gln Met Ala
65                  70                  75                  80

Gln Lys Ser Gln Ser Thr Gln Ile Ser Gln Glu Leu Glu Glu Leu Arg
                85                  90                  95

Ala Glu Gln Gln Arg Leu Lys Ser Gln Asp Leu Glu Leu Ser Trp Asn
            100                 105                 110

Leu Asn Gly Leu Gln Ala Asp Leu Ser Ser Phe Lys Ser Gln Glu Leu
        115                 120                 125

Asn Glu Arg Asn Glu Ala Ser Asp Leu Leu Glu Arg Leu Arg Glu Glu
    130                 135                 140

Val Thr Lys Leu Arg Met Glu Leu Gln Val Ser Ser Gly Phe Val Cys
145                 150                 155                 160

Asn Thr Cys Pro Glu Lys Trp Ile Asn Phe Gln Arg Lys Cys Tyr Tyr
                165                 170                 175

Phe Gly Lys Gly Thr Lys Gln Trp Val His Ala Arg Tyr Ala Cys Asp
        180                 185                 190

Asp Met Glu Gly Gln Leu Val Ser Ile His Ser Pro Glu Glu Gln Asp
        195                 200                 205

Phe Leu Thr Lys His Ala Ser His Thr Gly Ser Trp Ile Gly Leu Arg
    210                 215                 220

Asn Leu Asp Leu Lys Gly Glu Phe Ile Trp Val Asp Gly Ser His Val
225                 230                 235                 240

Asp Tyr Ser Asn Trp Ala Pro Gly Glu Pro Thr Ser Arg Ser Gln Gly
                245                 250                 255

Glu Asp Cys Val Met Met Arg Gly Ser Gly Arg Trp Asn Asp Ala Phe
        260                 265                 270

Cys Asp Arg Lys Leu Gly Ala Trp Val Cys Asp Arg Leu Ala Thr Cys
        275                 280                 285

Thr Pro Pro Ala Ser Glu Gly Ser Ala Glu Ser Met Gly Pro Asp Ser
    290                 295                 300

Arg Pro Asp Pro Asp Gly Arg Leu Pro Thr Pro Ser Ala Pro Leu His
305                 310                 315                 320

Ser

## Description

### TECHNICAL FIELD

The present invention relates to an antiarteriosclerotic agent and to a method of determining a condition of arteriosclerosis.

### BACKGROUND ART

Arteriosclerosis is a final stage of lifestyle-related diseases. No alternative methods exist for suppressing arteriosclerosis other than regulating the probability of development of this disease by controlling risk factors such as diabetes, high blood pressure, dyslipidemia, chronic kidney disease, and smoking (see Non-patent document 1), or, when acute catastrophic conditions (myocardial infarction, brain infarction, or critical limb ischemia) occur, lifesaving by fully utilizing medical resources (rescue and emergency systems, or acute hospitals with facilities and human resources that can administer an acute endovascular treatment). The strategy to control the risk factors lacks reliability so that its effectiveness is ultimately determined based on, for example, relative risk reduction derived from study populations in clinical trials.

The cause of this disease is considered to be a condition originating from inflammation and immune response along with a high-risk condition due to lifestyle-related diseases. The present inventors have reported that autoantibodies in serum against molecules such as IL-5 are associated with arteriosclerosis (see Non-Patent Document 2; and Patent Document 1). However, an aggressive therapy approach that acts on the cause of arteriosclerosis has not been developed.

Patent Document 2 discloses that depiction of B cells by an anti-CD20 antibody treats or prevents atherosclerosis. CD20, which is a 35 kDa nonglycosylated phosphoprotein found on the surface of more than 90% of B cells in peripheral blood or lymphoid organs, is expressed during early pre-B cell development, and persists until plasma cell differentiation (see Patent Document 2). Specifically, it is contemplated that anti-CD20 antibodies, when administered, target whole B cells to reduce the number of those cells.

### PRIOR ART DOCUMENT(S)

### PATENT DOCUMENT(S)

Patent Document 1: WO 2012/067165
Patent Document 2: JP 2012-514587 A

### NON-PATENT DOCUMENT(S)

Non-Patent Document 1: Anderson KM. et al., Am Heart J, 1991; 121:293-8
Non-Patent Document 2: Ishigami T. et al., FASEB J, 2013; 27:3437-3445

### SUMMARY OF THE INVENTION

### PROBLEMS TO BE SOLVED BY THE INVENTION

An object of the present invention is to provide a novel means that can suppress development of arteriosclerotic lesions and treat arteriosclerosis.

### MEANS FOR SOLVING THE PROBLEMS

Through extensive research using a mouse model of arteriosclerosis development, the present inventors have found that intestinal commensal microbiota promotes arteriosclerosis via activation of TLR signaling pathway in B2 cells, and that depletion of B2 cells in a mouse body by an anti-CD23 antibody suppresses development of arteriosclerosis. Moreover, the present inventors have further found that the expression level of particular genes, or the level of serum IgG3 or serum total IgG can be used as an indicator to determine development, and aggravation or improvement, etc. of arteriosclerosis, thereby completing the present invention.

Thus, the present invention provides an antiarteriosclerotic agent comprising, as an active ingredient, a substance which selectively suppresses B2 cells.

The present invention also provides a method of assisting in determining a condition of arteriosclerosis, comprising measuring expression, using a sample isolated from a subject, of at least one gene selected from the group consisting of the following gene group A and gene group B:
gene group A: Ccl2. Cd14, IIspa1a, Il1b, Il1r1, Ptgs2, Tnfrsf1a, Tlr5;
gene group B: Cebpb, Fos, Jun, Nfkbib, Chuk, Ikbkb, Irf1, Mapk8, Mapk8ip3, Mapk9, Tollip, Tradd.

The present invention further provides a method of assisting in determining a condition of arteriosclerosis, comprising measuring the level of IgG3 and/or the level of total IgG in a serum sample isolated from a subject.

### EFFECT OF THE INVENTION

A means capable of treating arteriosclerosis *per se* is provided by the present invention. Arteriosclerosis itself cannot be treated by improvement of lifestyle-related diseases, since the lifestyle-related diseases are not the root cause of arteriosclerosis but a risk thereof. In addition, endovascular treatment is a mere symptomatic therapy for acute thrombosis resulting from arteriosclerosis. The B2 cell-targeting antiarteriosclerotic agent of the present invention controls the cause of this disease to suppress development, progression and/or exacerbation of arteriosclerosis, which provides a therapy fundamentally different from the conventional treatment, thereby allowing significant contribution to extending healthy life expectancy. In development of arteriosclerosis, B2 cells are activated through lipid metabolism-independent mechanism, and when activation of B2 cells is suppressed, arteriosclerosis can be suppressed even in a condition with elevated serum lipid levels and high visceral and subcutaneous fat levels. Therefore, with the antiarteriosclerotic agent of the present invention, severe lipid restrictions imposed on arteriosclerosis patients may be relaxed. Furthermore, the method of determining a condition according to the present invention allows for properly assessing a condition of arteriosclerosis in a patient, as well as for monitoring a therapeutic effect of antiarteriosclerotic agents and determining a therapeutic effect of candidate antiarteriosclerotic agents, thereby also contributing to development of an additional therapy approach to arteriosclerosis.

### BRIEF DESCRIPTION OF THE DRAWINGS

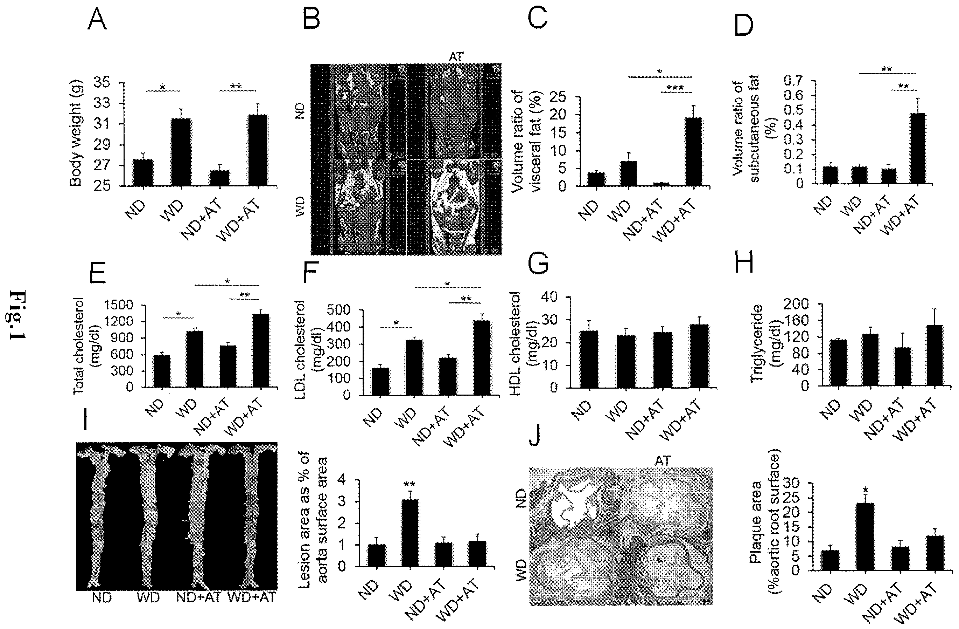

Fig. 1 shows data demonstrating that mice with antibiotic treatment are resistant to diet-induced atherosclerosis. Mice were given a Western diet or normal chow diet for 8 weeks with or without antibiotic treatment. (A) Body weights in each mouse group. AT: antibiotic treatment; WD: Western diet; ND: normal chow diet. Data were pooled from three independent experiments (means ± standard error, n = 7 for each group). (B-D) Representative micro-CT images of fat (B), percentages of visceral fat volume (C), and percentages of subcutaneous fat volume (D) are shown. Data are representative of means ± standard error from three independent experiments. n = 7 for each group. *p < 0.05, **p < 0.01, ***p < 0.001 according to the post-hoc ANOVA statistical analysis. (E-H) Serum levels of total cholesterol (E), LDL cholesterol (F), HDL cholesterol (G), and triglyceride (H) were assessed. LDL: low-density lipoprotein; HDL: high-density lipoprotein. Results are presented as means ± standard error. n = 7 for each group. *p < 0.05, **p < 0.001. (I, J) Aortic lesion size was assessed by oil red O staining (I) and HE staining (J). Representative images and quantitative results of oil red O staining of aortas prepared en face are shown in I (percentage of aortic surface area, n = 12 for each group). Representative images and quantitative results of maximum lesion area within the aortic root sections as determined by HE staining are shown in J (n = 12 for each group). Scale bars represent 100 µm. Data were pooled from three independent experiments. *p < 0.05, **p = 0.001, comparing the WD group with the other groups by ANOVA.

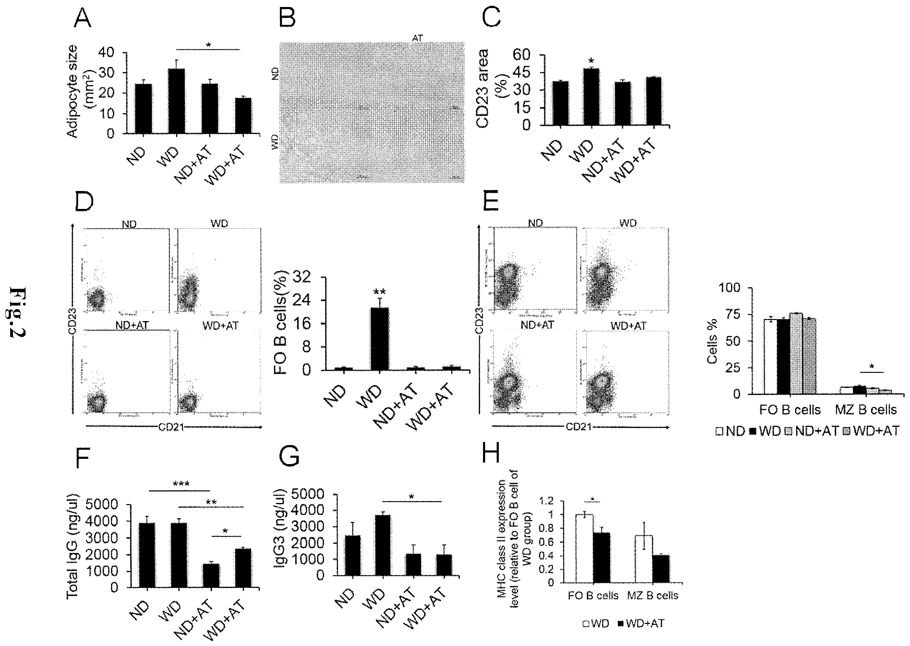

Fig. 2 shows data demonstrating that antibiotic treatment controls atherosclerosis by blocking B2-cell expansion. At the end of 8-week Western diet (WD) or normal chow diet (ND) feeding, the effects of antibiotic treatment (AT) on the typical features of perivascular adipose tissue (PVAT) expansion, and accumulation of CD23 positive cells are shown in A-C. (A) The mean adipocyte diameter was quantified in PVAT sections. The surface area of at least 250 adipocytes per image was determined, and a total of seven images per PVAT were analyzed with ImageJ software. Data represent means ± standard error from n =7 experiments. *p < 0.001 for one-way ANOVA with Tukey corrections. (B and C) Representative images of CD23-positive staining in PVAT sections (B) and quantitative results thereof (C) are shown. Scale bars represent 50 µm. Values are presented as means ± standard error. *p < 0.001, comparing the WD group with the other groups by ANOVA. (D and E) Flow cytometry analysis results of follicular (FO) B cells of PVAT. and flow cytometry analysis results of FO B cells and marginal zone (MZ) B cells in spleen are shown, respectively. n = 6 mice per group. Data were from 14 mice pooled from three independent experiments. Cell counts were presented as means ± standard error. *p < 0.01. **p < 0.001. (F and G) Concentrations of total IgG (F) and IgG3 (G) were assessed by ELISA. Values were presented as means ± standard error. n = 6 per group from three independent experiments. *p < 0.05, **p < 0.01, ***p < 0.001 by Tukey post-hoc test. (H) RNA expression of MHC class II was analyzed in purified, cell-sorted B220⁻CD21^{+/lo}CD23⁺ (FO B cells) and B220⁺CD21^{high}CD23^{-/lo} (MZ B cells) in spleen digests from WD and WD-AT groups by real-time PCR and normalized to GAPDH. Fold changes of 2^{ΔCt} were presented as means ± standard error. *p < 0.05. n = 7 per group.

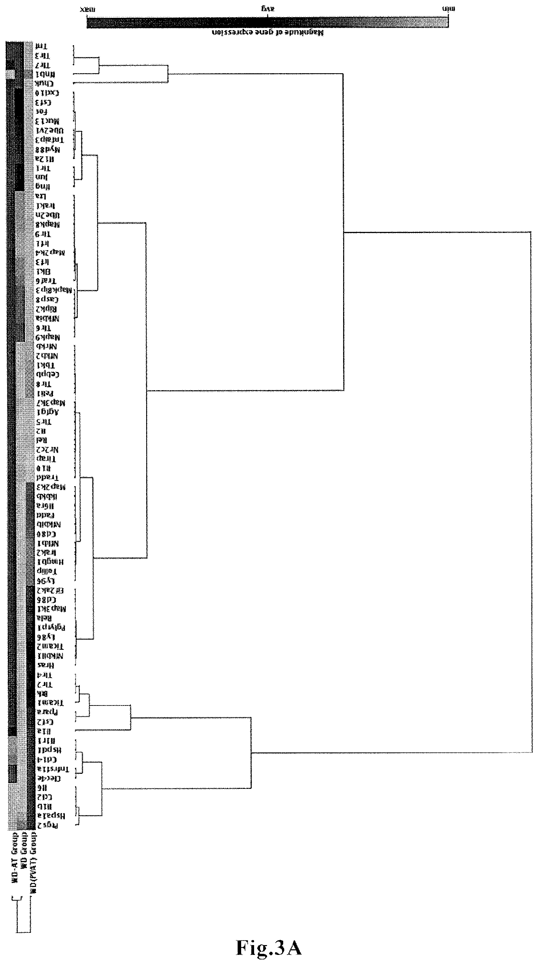

Fig. 3A shows a heat map representing gene expression profiles associated with TLR signaling pathway in B2 cells following WD feeding and AT. mRNA preparations of sorted FO B cells from PVAT and spleen were analyzed by mouse toll-like receptor signaling pathway RT2 Profiler PCR arrays. Expression in FO B2 cells of genes reportedly associated with TLR signaling pathway was compared among the indicated groups.

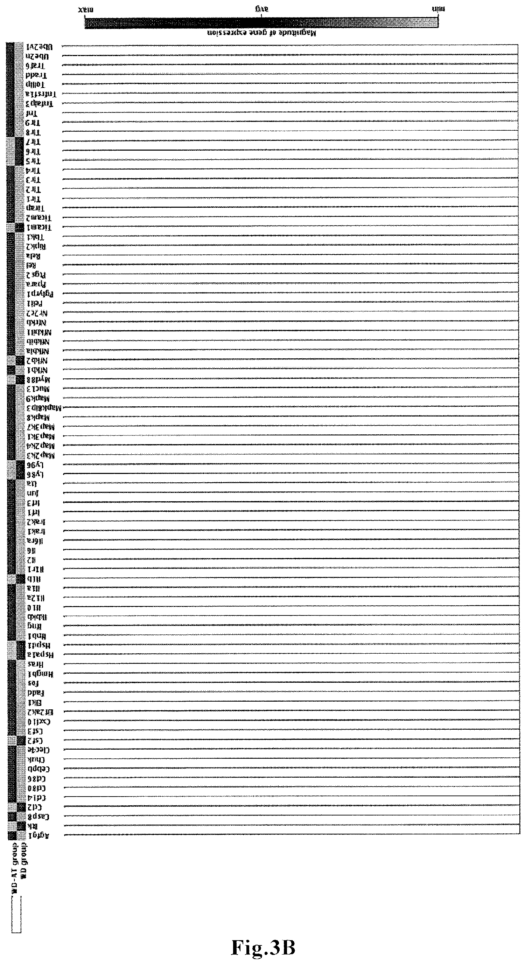

Fig. 3B shows a heat map representing gene expression profiles associated with TLR signaling pathway in B2 cells following WD feeding and AT. mRNA preparations of sorted MZ B cells from spleen were analyzed by mouse toll-like receptor signaling pathway RT2 Profiler PCR arrays. Expression in MZ B2 cells of genes reportedly associated with TLR signaling pathway was compared among the indicated groups.

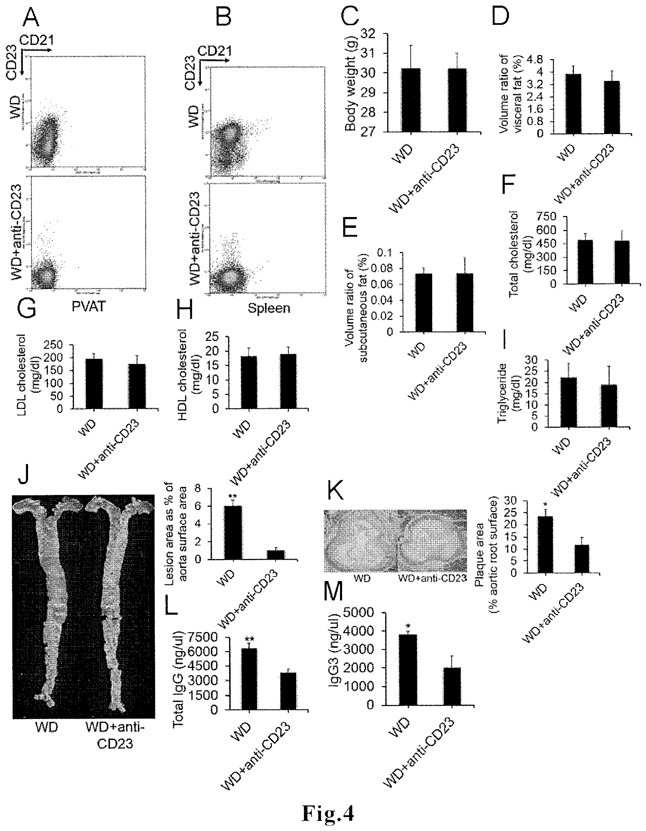

Fig. 4 shows data demonstrating that pharmacological depletion of B2 cells protects mice from atherosclerosis. (A and B) Representative flowcytometric plots of B2-cell numbers in the PVAT (A) and spleens (B) of mice treated with a mouse specific CD23 antibody or saline (n=6 per group) are shown. (C) Body weights were measured at the end of 8-week Western diet feeding in mice treated with a mouse specific CD23 antibody or saline. Values are presented as means ± standard error from n=6 experiments. (D and E) Percentages of visceral fat volume (D) and subcutaneous fat volume (E) were evaluated with Micro-CT. Data are presented as means ± standard error. n = 6 for each group. (F-I) Serum levels of total cholesterol (F), LDL cholesterol (G), HDL cholesterol (H), and triglyceride (I) were measured. Results are presented as means ± standard error. n = 6 per group. (J and K) The effect of an anti-mouse CD23 antibody on plaque formation is shown in J-K. (J) Representative images and quantitative results of oil red O staining of aortas prepared en face are shown. (K) Representative images of HE staining of aortic root sections and quantitative results of maximum lesion area are shown. Data (means ± standard error) were obtained from n = 6 experiments. Scale bars represent 100 µm. *p < 0.05, **p < 0.001 for one-way ANOVA with Tukey's corrections. (L and M) Concentrations of total IgG (L) and IgG3 (M) in serum from mice treated with a mouse-specific CD23 antibody or saline are shown. Values are presented as means ± standard error from n = 6 experiments. *p < 0.05, **p < 0.01 for one-way ANOVA with Tukey's corrections.

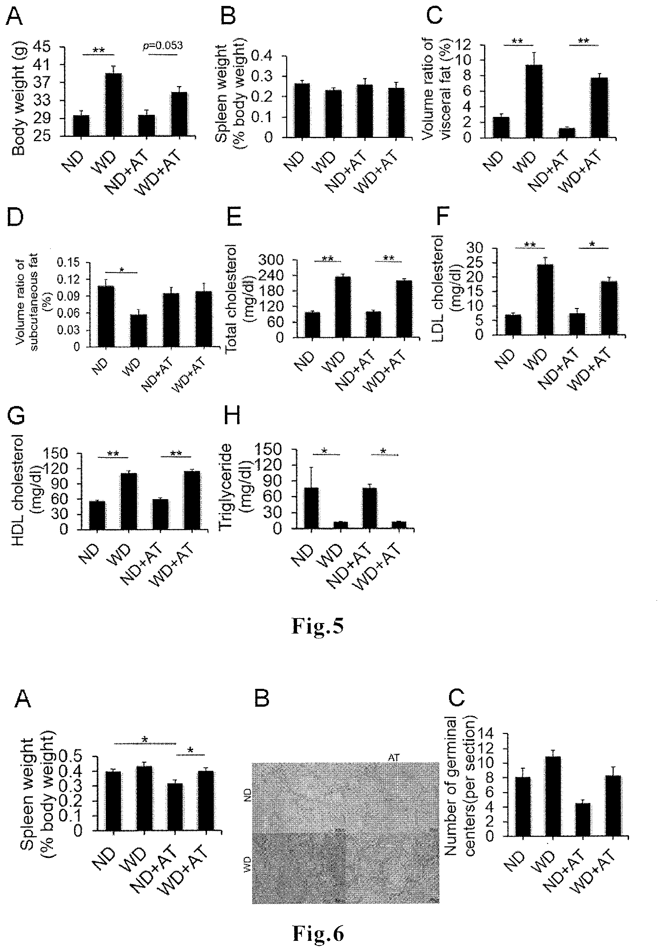

Fig. 5 shows characteristics of C57BL/6J wild type mice that received WD feeding and antibiotic treatment. (A) Mean body weights ± standard error in each mouse group. Significant differences were calculated by one-way ANOVA. **p < 0.001. n = 5 for each group. (B) Spleen weights were expressed as a percentage of body weight (%). Results are presented as means ± standard error. n = 5 for each group. (C and D) Percentages of visceral fat volume (C) and subcutaneous fat volume (D) were determined using micro-CT. n = 5 for each group. *p < 0.05, **p < 0.001 according to the post-hoc ANOVA analysis. (E-H) Serum levels of total cholesterol (E), LDL cholesterol (F), HDL cholesterol (G), and triglyceride (H) are shown. n = 5 for each group. *p < 0.01, **p < 0.001.
Fig. 6 shows typical features of spleen in each group. (A) Spleen weights were measured at the end of the study. Data were expressed as a percentage of body weight. (B and C) Spleen sections were stained with HE (B), and the number of germinal centers was counted (C). Scale bars represent 300 µm. Statistical significance was calculated by one-way ANOVA. *p < 0.05. n = 7 for each group.

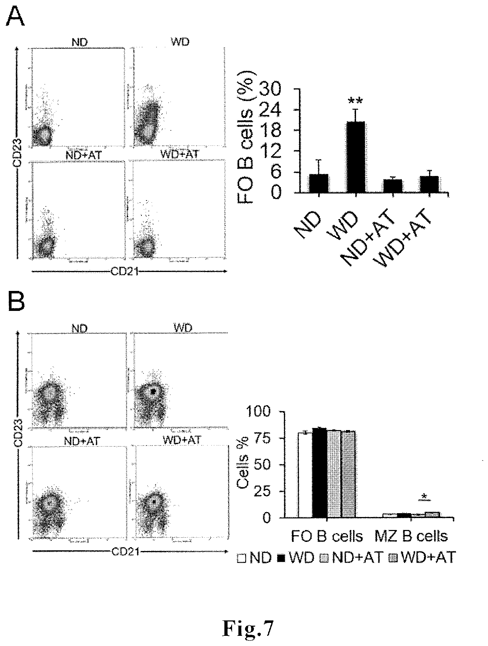

Fig. 7 shows the effects of antibiotic treatment on B2-cell numbers in the PVAT and spleen of C57BL/6J wild type mice. (A) Flow cytometry results of FO B cells in PVAT, and (B) flow cytometry results of FO B and MZ B cells in spleen. n = 5 for each group. The graphs represent cell counts (mean ± standard error). *p < 0.05, **p < 0.01.

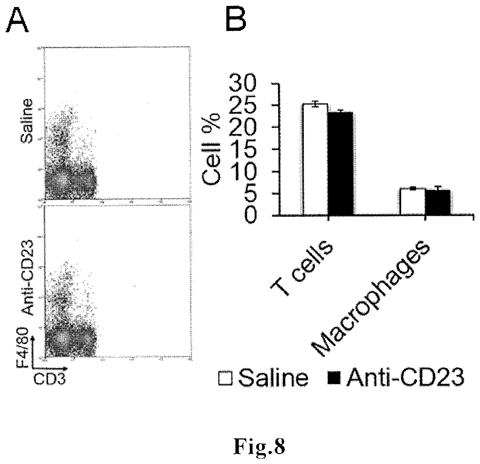

Fig. 8 shows the effects of an anti-mouse CD23 antibody on the numbers of T cells and macrophages in spleen. (A and B) The total numbers of T cells and macrophages in the spleen of the mice that received saline or mouse-specific CD23 antibody. (A) Representative flow cytometry plots, and (B) data of the groups. Numerical values are presented as means ± standard error from n=6 experiments.

### MODE FOR CARRYING OUT THE INVENTION

An antiarteriosclerotic agent according to the present invention comprises, as an active ingredient, a substance which selectively suppresses B2 cells. The term "suppressing B2 cells" encompasses suppressing activation of B2 cells, for example, reducing the number of detectable B2 cells. A substance which selectively suppresses B2 cells is a substance which suppresses B2 cells and does not suppress B1 cells and other B-cell subsets. For example, if a living body systemically treated with a certain substance maintains the number of B1 cells in the spleen at 65% or more (e.g. 70% or more, 75% or more, 80% or more, 85% or more, or 90% or more) of the number of B1 cells in the spleen of an untreated body, and reduces the number of B2 cells in the spleen at less than 20% (e.g. less than 15%, less than 10%, less than 7%, or less than 5%) of the number of B2 cells in the spleen of an untreated body, the substance falls into "a substance which selectively suppresses B2 cells". Arteriosclerosis can be treated by selectively suppressing B2 cells, as shown specifically in the following Examples. The term "treating arteriosclerosis" encompasses suppressing progression and/or exacerbation of arteriosclerotic lesions, and suppressing development of new arteriosclerotic lesions.

Whether a certain substance has an action of suppressing B2 cells or not can be determined, for example, by treating a peripheral blood sample with the substance, and subsequently measuring the number of B2 cells by flow cytometry, followed by comparing it with the number of B2 cells in a peripheral blood control sample untreated with the substance.

Typical examples of the substance which selectively suppresses B2 cells include an anti-CD23 antibody. CD23, which is a low-affinity IgE receptor, is a surface antigen highly specific for B2 cells among B cells, such that an anti-CD23 antibody can be used to selectively suppress B2 cells *in vivo.*

A variety of anti-CD23 antibodies are known and commercially available. Anti-human CD23 monoclonal antibodies available for administration to humans are also already known (e.g. WO 98/37099). Amino acid sequences of CD23 of various animal species including human, and cDNA sequences encoding the amino acid sequences are also known and registered in a database. Thus, an anti-CD23 antibody can also be produced in a conventional manner. For example, human CD23 is registered under accession numbers such as NM 002002 in the NCBI GenBank. Sequences shown in SEQ ID NO:3 and SEQ ID NO:4 in the sequence listing are sequences of human CD23 registered under NM_002002.4.

An anti-human CD23 monoclonal antibody may be produced by the hybridoma method, for example, by immunizing a non-human animal with human CD23 protein or an appropriate fragment thereof (an extracellular domain such as aa48-321 of human CD23) as an immunogen: collecting antibody-producing cells such as splenic cells or lymphocytes from the immunized animal; preparing hybridomas by fusing the collected cells with myeloma cells; and screening the hybridomas for production of an antibody that binds to CD23 protein. The hybridoma selected by the screening can be grown to obtain an anti-human CD23 monoclonal antibody from the culture supernatant.

In the case of using an anti-CD23 antibody as a human pharmaceutical, it is preferable to use an antibody that has reduced immunogenicity to humans, such as a human chimeric antibody (typically, a rodent-human or primate-human chimeric antibody), a humanized antibody (an antibody prepared by grafting the CDR regions of a non-human-derived antibody into the corresponding regions of a human antibody), or a human antibody (the same antibody as an antibody produced in a human body, which is prepared using a non-human animal or a human cell line). The methods of producing a chimeric antibody, a humanized antibody, and a human antibody are also well-known and well-established.

The term an anti-CD23 antibody encompasses antibodies that have various activities such as a neutralizing activity (neutralizing, blocking, or inhibiting), ADCC activity, and CDC activity. In the present invention, an anti-CD23 antibody can have any of such activities as long as they can suppress B2 cells.

In the present invention, arteriosclerosis is typically atherosclerosis.

A subject to whom the antiartcriosclerotic agent of the present invention is administered includes various mammals, and is typically human. The antiarteriosclerotic agent of the present invention can be preferably administered to a patient, typically a human patient, with arteriosclerosis.

The dosage of the antiarteriosclerotic agent may be the amount sufficient to treat arteriosclerosis, and appropriately selected depending on, for example, the age, body weight, and severity of a subject who receives it. The dosage may be, as an active ingredient amount per day, from about 1.5 µg/kg body weight to about 1.5 g/kg body weight, for example, from about 10 µg/kg body weight to about 100 mg/kg body weight for the subject, but not restricted thereto. The above-described dosage may be given to a subject once or dividedly in a few or several times in a day. The agent may be administered daily, or every few or several days, or every few or several weeks.

The route of administration of the antiarteriosclerotic agent may be oral or parenteral, and in general, preferably parenteral such as intramuscular, subcutaneous, intravenous, or intraarterial administration. Although systemic administration is typically preferred, the antiarteriosclerotic agent may also be administered locally to the vicinity of the arteriosclerotic lesion.

Dosage forms of the antiarteriosclerotic agent are not particularly limited, and, depending on the route of administration, can be formulated by appropriately mixing additives such as pharmaceutically acceptable carriers, diluents, or excipients with the above-described active ingredient. Examples of the formulations include oral formulations such as tablets, capsules, granules, powders, or syrups; and parenteral formulations such as inhalants, injections, suppositories, or solutions. Formulation methods and available additives are well-known in the field of the pharmaceutical formulation, and any methods and additives can be used.

The present invention also provides a method of assisting in determining a condition of arteriosclerosis. A first aspect of the method comprises measuring expression of at least one gene selected from the group consisting of the following gene group A and gene group B using a sample isolated from a subject.

Gene group A:
   Ccl2 (UniGene Hs.303649, Refseq NM_002982.3)
   Cd14 (UniGene Hs.163867, Refseq NM_000591.3)
   Hspa1a (UniGene Hs.274402, Refseq NM_005345.5)
   Il1b (UniGene Hs.126256, Refseq NM_000576.2)
   Il1r1 (UniGene Hs.701982, Refseq NM_000877.2)
   Ptgs2 (UniGene Hs.196384, Refseq NM_000963.2)
   Tnfrsf1a (UniGene IIs.279594, Refseq NM 001065.3)
   Tlr5 (UniGene Hs.604542, Refseq NM_003268.5).
Gene group B:
   Cebpb (UniGene Hs.719041, Refseq NM_005194.3)
   Fos (UniGene Hs.25647, Refseq NM_005252.3)
   Jun (UniGene Hs.696684, Refseq NM_002228.3)
   Nfkbib (UniGene Hs.9731, Refseq NM_002503.4)
   Chuk (UniGene Hs.198998. Refseq NM_001278.3)
   Ikbkb (UniGene Hs.597664, Refseq NM_001556.2)
   Irf1 (UniGene Hs.436061. Refseq NM_002198.2)
   Mapk8 (UniGene Hs.138211, Refseq NM_002750.2)
   Mapk8ip3 (UniGene Hs.207763, Refseq NM_015133.3)
   Mapk9 (UniGene Hs.484371, Refseq NM_002752.4)
   Tollip (UniGene Hs.368527, Refseq NM_019009.3)
   Tradd (UniGene Hs.460996, Refseq NM_003789.3).

In the present invention, the term "determining a condition" encompasses determining the presence or absence of arteriosclerosis, exacerbation/severe progression thereof, and mitigation/improvement thereof.

In the present invention, a subject is typically a patient with arteriosclerosis.

A sample used in the first aspect is not particularly limited, as far as the sample contains B cells, especially B2 cells. For example, peripheral blood may be collected from the subject, and the monocytic cell fraction may be concentrated and isolated from the peripheral blood by conventional cell sorting using appropriate surface markers. Thereafter, the obtained cell fraction may be examined for the expression of at least any one of the genes listed in the above-described gene groups.

The genes belonging to the gene group A are the genes whose expression level has been confirmed to be higher in an individual with development of arteriosclerosis than in an individual with suppressed development of arteriosclerosis. If high expression of a gene(s) belonging to the gene group A is detected, it can be determined that arteriosclerosis has been developed or exacerbated in the subject. By contrast, if low expression of a gene(s) belonging to the gene group A is detected, it can be determined that arteriosclerosis is not developed in the subject. In addition, if the expression level of a gene(s) in the gene group A is lowered in a patient with arteriosclerosis compared to the previously-detected expression level of the gene in the same patient, it can be determined that arteriosclerosis has been ameliorated in the patient.

The genes belonging to the gene group B are the genes whose expression level has been confirmed to be lower in an individual with development of arteriosclerosis than in an individual with suppressed development of arteriosclerosis. If low expression of a gene(s) belonging to the gene group B is detected, it can be determined that arteriosclerosis has been developed or exacerbated in the subject. By contrast, if high expression of a gene(s) belonging to the gene group B is detected, it can be determined that arteriosclerosis is not developed in the subject. In addition, if the expression level of a gene(s) in the gene group B is increased in a patient with arteriosclerosis compared to the previously-detected expression level of the gene in the same patient, it can be determined that arteriosclerosis has been ameliorated in the patient.

High expression as used herein refers to a situation where the expression level is higher than a healthy reference value determined based on expression levels in a healthy population (e.g. mean of expression levels in respective healthy individuals) or where the expression level is higher than the expression level previously measured in the same subject. Meanwhile, low expression refers to a situation where the expression level is lower than the healthy reference value or where the expression level is lower than the expression level previously measured in the same subject.

Measurement of gene expression encompasses measurement of the mRNA expression level and measurement of the protein expression level. The mRNA expression level can be measured by a conventional method such as real-time PCR, microarrays, or Northern blotting. The protein expression level can be measured by a conventional method such as immunoassay.

A second aspect of the method of determining a condition comprises measuring the level of IgG3 and/or the level of total IgG in a serum sample isolated from a subject. As shown in the following EXAMPLES, the serum IgG3 level is higher in a group of individuals with arteriosclerosis, and lower in a group of healthy individuals without arteriosclerosis and in a group of individuals in which arteriosclerosis is suppressed by, e.g., administration of an anti-CD23 antibody. This variation in the serum IgG3 level can also be seen as a variation in the serum total IgG level. Thus, the condition of arteriosclerosis can be determined based on the level of IgG3 or total IgG in a serum sample. Either one or both of the levels of IgG3 and total IgG in a serum sample may be measured.

If a high level(s) of serum IgG3 and/or total IgG is/are detected, it can be determined that arteriosclerosis has been developed or exacerbated in the subject. In addition, if the level(s) of serum IgG3 and/or total IgG is/are lowered in a patient with arteriosclerosis compared to the previously-measured level(s) of serum IgG3 and/or total IgG in the same patient, it can be determined that arteriosclerosis has been ameliorated in the patient.

The high level of serum IgG3 as used herein refers to a situation where the serum IgG3 level is higher than a healthy reference value determined based on serum IgG3 levels in a healthy population (e.g. mean of serum IgG3 levels in respective healthy individuals) or where the serum IgG3 level is higher than the serum IgG3 level previously measured in the same subject. This definition is also applied to the term "high level of serum total IgG".

The levels of serum IgG3 and serum total IgG can be measured by a conventional method such as immunoassay. A kit such as an ELISA kit that can measure serum IgG3 or serum total IgG in various animals including humans and mice is commercially available, and such commercially available products may also be used.

### EXAMPLES

The present invention will now be described in more detail by way of examples. It should be noted, however, that the present invention is not limited to the following examples.

### <Materials and Methods>

### Mice

ApoE knockout (KO) mice, known mice on the C57BL/6J background (Lo Sasso et al., J Transl Med 2016, 14:146, DOI 10.1186/s12967-016-0901-1; Kapourchali et al., World J Clin Cases 2014 May 16, vol. 2, issue 5, p.126-132; Li et al., Protein Cell 2011, vol. 2, issue 3, p.189-201), were used. C57BL/6J mice were purchased from Oriental Yeast (Tokyo, Japan). The number of mice per group used in each experiment is annotated in the corresponding figure legend as n. All mice admitted to the study survived the intended duration of the study, and only 5-week-old male mice were used for experiments. Until required for the experiments, the mice were provided with a standard diet and tap water ad libitum. All of the animal studies were conducted in accordance with the animal care guidelines of Yokohama City University Graduate School of Medicine.

### Commensal Bacteria Depletion

The role of microbiota was investigated by treating mice with antibiotics. For experiments, 5-wcek-old male ApoE KO mice were assigned to a control group or an antibiotic treatment (AT) group. Mice in the AT group were treated with a broad-spectrum antibiotic cocktail AVNM. which contained 0.5 g/L vancomycin, 1 g/L neomycin sulfate, 1 g/L metronidazole, and 1 g/L ampicillin. The antibiotic cocktail was provided in drinking water for one week before beginning a Western diet and continued during the experimental period (Croswell et al., 2009; Rakoff-Nahoum et al., 2004; Wang et al., 2011). Antibiotics were purchased from Sigma or Wako (Tokyo, Japan). The drinking water containing antibiotics was changed every 3-4 days.

### Active Induction of Atherosclerosis

One week after the antibiotic treatment, each mouse was provided with a Western diet (WD; containing 21.22% [g/100 g] fat, 17.01% protein, 48.48% carbohydrate, and 0.15% cholesterol; Oriental Yeast) or a normal chow diet (ND), and was maintained on the diet for 8 weeks.

### Micro-CT

Micro-CT scanning was performed to evaluate visceral and subcutaneous fat before sacrifice. Anesthetized mice were imaged on the in vivo micro X-ray CT system R_mCT2 (Rigaku Co., Tokyo. Japan) (scan conditions: voltage, 90 kV; current, 160 µA; FOV, 60 mm; scanning time, 17 s). Whole visceral and subcutaneous fat volumes were traced. Quantitative measurements were performed using Metabolic Analysis Software (Rigaku Co.).

### Quantification of Atherosclerosis and Histologic Analysis

After 8 weeks of consuming a Western diet, hearts and aortas were removed, and atherosclerotic lesions were quantified by cross-sectional analysis of the aortic root and by en face analysis of the whole aorta. The hearts were embedded in paraffin, and serial 5 µm sections were cut from the beginning of the three aortic leaflets to the ascending aorta and then stained with hematoxylin and eosin (H&E) stain. Afterwards, the aortas were fixed in 10% formalin, opened longitudinally, and stained for lipid depositions with Oil Red O solution (Sigma-Aldrich, USA). Lesion areas were calculated using the Image J program (National Institutes of Health, USA; http://rsb.info.nih.gov/ij). For plaque measurements, sections that captured the maximum lesion area were used to compare lesion sizes between study groups. Plaque areas and total blood vessel area were determined, and the relative plaque extension was expressed as a percentage of the total vessel area.

### Histologic and Immunohistochemical Analysis

Freshly isolated spleens were weighed, imaged and then formalin-fixed, paraffin-embedded, and sectioned in a thickness of 5 µm. Spleen architecture was evaluated after H&E staining. Numbers of germinal centers were assessed using Image J software. Tissue samples from perivascular adipose tissue (PVAT) were processed and embedded in paraffin according to standard procedures. Sections of 5 µm were stained with H&E stain or CD23 antibody (Product code: ab185807, Abcam, USA). Fat cell areas on H&E-stained slides were measured using Image J software, quantifying two different tissue sections per mouse (at least 3 mice with at least 100 fat cells in each image). In addition, CD23 antibody specific for B2 cells was used to stain B2 cells in PVAT. The percentage area of CD23 positive field was assessed in a blinded manner using Image J software.

### Preparation of Tissues for Flow Cytometry and Cell Sorting

Splenocytes and PVAT cells were harvested and processed for flow cytometry. In brief, cells were released from spleen by nicking the capsule, and two microscope slides were gently rotated. Remaining erythrocytes were removed by incubation with a red blood cell lysing buffer (Product No. R7757, Sigma). Meanwhile, complete PVAT cells were freed from aortas at 4 x magnification and digested by collagenase type 4 (0.5 mg/mL) (Catalog No. LS004186, Worthington Biochemicals) for 30 min at 37°C; total PVAT cells were then collected. Single-cell suspensions were filtered through a nylon mesh (100 µm), BD Biosciences) and stained either with a mixture of fluorochrome-labeled anti-mouse monoclonal anti-CD21 antibody (APC-labeled, Catalog No. 561770, BD Biosciences), anti-CD23 antibody (PE-labeled, Catalog No. 561773, BD Biosciences), anti-CD45R/B220 antibody (FITC-labeled, Catalog No. 561877, BD Biosciences) or anti-CD45 antibody (FITC-labeled. Catalog No. 561088, BD Biosciences), anti-F4/80 antibody (PE-labeled, Catalog No. 565410, BD Biosciences), anti-CD3e antibody (APC-labeled, Catalog No. 557306, BD Biosciences) for 30 min on ice. Then, the cells were resuspended in a fixable 4',6-diamidino-2-phenylindole (DAPI, Catalog No. P4864, Sigma) live/dead stain diluted with flow cytometry staining buffer (Catalog No. 00-4222-57, eBioscience). Flowcytometric analyses were performed on a MoFlo Astrios (Beckman Coulter), and the results were analyzed by software ver. 6.2.3. Dead cells were excluded by FSC, SSC, and DAPI staining. Distinct B-cell populations, T cells, and macrophages were identified by their different cell surface antigen expression. Specifically, FO B cells were identified as B220⁺CD21^{+/lo}CD23⁺ cells, MZ B cells were identified as B220⁺CD21^{high}CD23^{+/lo} cells, transitional B cells were identified as B220⁺CD21⁻CD23⁻ cells, and B1 cells were identified as B220^{low}CD23⁻ cells. T cells were identified as CD45⁻CD3e⁺F4/80⁻ cells, and macrophages were identified as CD45⁺F4/80⁺CD3e⁻ cells. For cell sorting experiments, the FO B and MZ B cells were sorted to higher than 99% purity from their parent gate.

### Real-time PCR (RT-PCR) Analysis

Total RNA was isolated from the pooled sorted cells using RiboPure Kit (Catalog No. AM 1924, Life Technologies) or RNeasy Plus Micro Kit (Catalog No. 74034, QIAGEN) according to the manufacturer's protocol. Using a High Capacity RNA-to-cDNA Kit (Catalog No. 4387406, Applied Biosystems) according to the manufacturer's protocol, 1 µg of RNA was reverse-transcribed to cDNA. The expression levels of major histocompatibility complex (MHC)-class II were analyzed by RT-PCR using SYBR-Green (Catalog No. 4309155, Applied Biosystems) on an ABI 7500 RT-PCR machine according to the manufacturers' recommendations. The relative mRNA expression levels were determined by the 2[-ΔΔC (T)] method, using glyceraldehyde-3-phosphate dehydrogenase (GAPDH) as a housekeeping gene. The following primers were used: mouse MHC-class II (LeibundGut-Landmann et al., 2004): I-Aa forward, 5'-AAATTCCACCCCAGCTACCAAT-3' (SEQ ID NO:1); I-Aa reverse, 5'-GCTGACCCAGCAGCACAG-3' (SEQ ID NO:2). To analyze toll-like receptor (TLR) signaling genes, mouse TLR signaling pathway RT2 Profiler PCR arrays (Catalog No. PAMM-018Z, Qiagen) were used to profile the expression of 84 genes related to TLR signaling. Total RNA from pooled sorted cells was used, and single-stranded cDNAs were synthesized from 25 ng of total RNA by using an RT2 PreAMP cDNA synthesis kit and RT2 PreAMP Pathway Primer Mix (Catalog No. 330451; PBM-018Z, Qiagen). The cDNAs were mixed with RT2 SYBR Green ROX qPCR Mastermix, and RT-PCR was performed in accordance with the manufacturer's instructions. Thermal cycling and fluorescence detection were performed using an ABI 7500 Sequence Detection System (Applied Biosystems). Data were quantified using an online software, RT2 Profiler PCR Array Data Analysis version 3.5 (Qiagen, http://perdataanalysis.Sabiosciences.Com/per/arrayanalysis.php?target=upload). Only Ct values < 35 were included in the calculations, and gene expression was evaluated relative to the mean expression of β2 microglobulin (β2M) and GAPDH, known as housekeeping genes. β2M and GAPDH were used since these were the two most stable of the five housekeeping genes included in the array. The transcripts of interest were reported as log2 fold changes and average expression levels, and the expressions of TLR-regulated transcripts were compared between the groups as heat maps.

### Quantification of Serum Parameters

Whole blood was harvested from mice at the time of sacrifice by right ventricular puncture. Total cholesterol, low-density lipoprotein cholesterol (LDL-C), high-density lipoprotein cholesterol (HDL-C), and triglyceride were determined by SRL Inc. with LDL cholesterol Kit (Catalog No. 30173000, Sekisui Medical Co. Ltd., Tokyo, Japan), HDL-cholesterol Kit (Catalog No. 30169000, Sekisui Medical Co. Ltd.), Cholesterol Kit (Catalog No. 460-44301, Wako Pure Chemical Co., Tokyo, Japan), and Triglyceride Kit (Catalog No. 464-44201, Wako Pure Chemical Co.) (Hashimoto et al.. 2006; Hosoi et al., 2010; Irie et al., 2015). LDL-cholesterol Kit and HDL-cholesterol Kit described above are the credible assay kits of LDL-C and HDL-C, and have been certified through the certification program of the Centers for Disease Control and Prevention (CDC) and Cholesterol Reference Method Laboratory Network (CRMLN). Immunoglobulin measurement: serum total IgG and IgG3 were measured in diluted serum with the Mouse IgG ELISA Kit (Bethyl Laboratories, E99-131) and Mouse IgG3 ELISA Kit (Bethyl Laboratories, E99-111) according to the manufacturer's instructions. Data were expressed as µg/mL, based on the standard curves of isotype standards.

### Depletion of B2 Cells by Antibody Treatment

To determine the possible involvement of commensal microbe-specific activation of B2-cell subsets in atherogenesis development, we administered a neutralizing anti-CD23 antibody (Purified Rat Anti-Mouse CD23, BD Pharmingen. B3B4, Isotype IgG2a) 50 µg i.p. (0.5 µg/µL; 100 µL) one week prior to starting the Western diet feeding and then 50 µg i.p. each week during the induction οf atherosclerosis, thereby ablating B2 cells (Coyle et al.. 1996; Dasic et al., 1999). The control group received saline instead. The rat anti-CD23 monoclonal antibody B3B4 used herein have been reported to block the binding of IgE to CD23 in vivo and in vitro (Rao M, et al. 1987. J. Immunol. 138:1845.; Oshiba A, et al. 1997. J. Immunol. 159:4056.; Dasic G, et al. 1999. Eur. J. Immunol. 29:2957).

### Statistical Analysis

Statistical analyses were performed with the SPSS 22 software program. Differences between groups were considered to be statistically significant when p < 0.05 using the appropriate tests. We used one-way ANOVA tests followed by Tukey post-hoc tests when values were normally distributed or nonparametric Kruskal-Wallis tests when values were not normally distributed.

### <Results>

### Intestinal Commensal Microbiota Promotes the Development of Atherosclerosis

To assess the impact of intestinal commensal microbiota on atherosclerosis, the intestinal commensal microbiota was eliminated by antibiotic treatment. Although we did not perform bacterial culture of feces or RT-PCR amplification of 16S rRNA gene sequences to quantitatively analyze the total biota, the results of previous studies indicate that a broad-spectrum antibiotic cocktail (AVNM) has sufficient activity to deplete all detectable commensal bacteria (Rakoff-Nahoum et al., 2004; Wang et al., 2011), and macroscopic examination of the gastrointestinal tract revealed characteristic secondary findings associated with the depletion of intestinal commensal microbiota, such as marked swelling of the cecum ascribed to the accumulation of undigested mucus (Barthel et al., 2003; Croswell et al., 2009). These findings indicate a reduction in the levels of colonizing bacteria (data not shown). The body weights in the Western diet (WD) and normal diet (ND) groups with or without antibiotic treatment (AT) were monitored weekly. The results of body weight measurements are shown in Fig. 1A. There was a significant difference in body weight between the WD and ND groups (p < 0.05); however, there was no obvious difference in body weight between the WD-fed mice and the age-matched WD-fed AT mice. This finding indicates that elimination of the intestinal microbiota by antibiotic treatment has no impact on WD-induced weight gain. Intriguingly, antibiotic treatment resulted in increased visceral and subcutaneous fat during WD feeding, whereas micro-CT analysis at the end of the 8-week diet study showed no changes in visceral or subcutaneous fat in WD-fed ApoE KO mice as compared with ND-fed ApoE KO mice. However, after antibiotic treatment, significant increases in visceral fat (p < 0.05) and subcutaneous fat (p < 0.01) were seen only in WD-fed mice (Fig. 1B-1D). The increased visceral and subcutaneous fat gain observed in the WD-AT mice prompted us to test whether this phenomenon was accompanied by metabolic alterations. As a result, we detected dyslipidemia in the WD-AT mice. The serum lipid levels increased significantly in the WD-AT mice, and the serum total cholesterol and LDL-C levels were significantly higher in the WD-AT mice than in the WD-fed mice (p < 0.05) (Fig. 1E-1H). Although the changes in the plasma cholesterol levels were obvious, arteriosclerotic lesion size, which was determined by oil red O staining of whole aortas after 8-week WD feeding, indicated that antibiotic treatment significantly decreased en face arteriosclerotic lesion size associated with a WD (p < 0.001) (Fig. 1I). Histological examination of the aortic roots confirmed a similar decrease in arteriosclerotic lesion size (p < 0.05) (Fig. 1J), as well as in macrophage and T cell-rich areas (data not shown). To assess whether these phenotypes were specific to ApoE KO mice, the same analyses were performed in normal C57BL/6J mice, and as expected similar results were obtained (Fig. 5). Analysis of the relation between plaque sizes and total cholesterol levels showed no correlation between these variables. This finding indicates that proatherogenic effects are related to intestinal commensal microbiota and do not solely depend on hypercholesterolemia. Collectively, these data suggest that intestinal commensal microbiota can promote arteriosclerosis development through lipid metabolism-independent mechanism.

### Antibiotic Treatment Reduces Atherosclerosis by Abolishing the WD-induced Increase in FO B Cells

B cells are known to reside also in the adventitial layer of vessels as well as in spleen (Grabner et al., 2009; Hamze et al., 2013). On the other hand, the role of gut microbes in regulating the immune system through metabolism-independent pathways has been described (Brown and Hazen, 2015; Drosos et al., 2015). Therefore, to test the hypothesis that the impact of intestinal microbiota on atherosclerosis is via activation of B2 cells, we initially analyzed B2-cell populations in spleen and PVAT.

In healthy individuals, adipose tissue expansion occurs by enlargement of the fat pad mass through enhanced recruitment of adipocyte precursor cells that differentiate into small adipocytes. In contrast, pathological expansion of adipose tissue is characterized by rapid growth of the fat pad through enlargement of existing fat cells. Such pathological expansion is associated with chronic inflammation (Sun et al., 2011). Therefore, the metabolic syndrome observed in WD-fed and WD-AT mice indicates that pathological adipose tissue expansion may occur in these mice. As expected, adipocytes in the PVAT of WD-fed mice were larger, less organized, and more loosely packed as compared with adipocytes in the control group (p < 0.001; Fig. 2A-2B). However, as shown in Fig. 2A-2B, typical features of adipocytes in the PVAT of WD-AT mice did not significantly differ from those in ND-fed mice. Moreover, we examined the effect of the elimination of intestinal commensal microbiota on the accumulation of B2 cells in the PVAT of WD-fed mice. Consistent with the above finding, WD-fed mice showed a significant increase in the accumulation of CD23-positive cells as assessed by immunohistochemical analysis (p < 0.001; Fig. 2B-2C). The numbers of CD23-positive cells were equivalent in the WD-AT and control groups (Fig. 2B-2C). This result suggests that pronounced B2 cell infiltration was augmented in the PVAT of WD-fed mice and completely abolished along with the depletion of all detectable commensal microorganisms. These findings support the notion that accumulated B2 cells are essential contributors to the induction of atherosclerosis by intestinal microbiota.

To further confirm the above findings, we studied the B2 cells in PVAT by flow cytometry. Representative flow cytometry plots are depicted in Fig. 2D. In the PVAT, the proportion of activated FO B cells dramatically increased in the WD-fed mice as compared with ND-fed mice (p < 0.001). However, after microbiota depletion by antibiotic treatment, the WD-induced increase in FO B cells was abolished (p < 0.001), which was consistent with the above finding. Concomitantly, we also investigated B2 cells in the spleen, the major B cell reservoir. Although there was no significant difference in spleen weight (Fig. 6A), and WD-feeding and antibiotic treatment did not modify the overall percentage of FO B cells in spleen (Fig. 2E) and was not associated with changes in splenic architecture indicative of FO B cell activation (Fig. 6B-6C), the proportion of MZ B cells was significantly decreased in the WD-fed mice after antibiotic treatment (p < 0.01; Fig. 2E). The above results were also confirmed in normal C57BL/6J mice (Fig. 7). Remarkably, the numbers of activated FO B cells were expanded in the PVAT of WD-fed mice, whereas AVNM-mediated microbiota depletion led to a significant reduction in FO B2 cells, and the expansion of the numbers of the FO B cells was completely abrogated in C57BL/6J mice (Fig. 7). As the primary function of activated B cells is to produce antibodies, we also investigated whether antibiotic treatment alters circulating IgG titers (Fig. 2F-2G). Although the increased number of FO B cells was not associated with increased total IgG titers in the serum of the WD-fed mice as compared with the ND-fed mice, antibiotic treatment sharply reduced the total IgG titers in the serum, followed by reductions in FO B cells and MZ B cells (p < 0.01; Fig. 2F). Moreover, analyses of IgG3, which is a specific subclass of IgG and mainly produced by MZ B cells via a T cell-independent pathway under preimmune conditions or after immunization (Guinamard et al., 2000; Panda et al., 2013), revealed that WD-AT mice displayed decreased serum levels of IgG3 as compared with WD-fed mice (p < 0.05; Fig. 2G). Moreover, there was also a trend toward increased serum levels of IgG3 in WD-fed mice relative to the ND-fed mice, although the difference did not reach statistical significance. We further examined the mRNA expression levels of MHC-class II in B2 cells, which can mediate antigen presentation and represent another important function of activated B cells. Although there was no difference in percentages of spleen FO B2 cells between the WD group and the WD-AT group on flow cytometric analysis (Fig. 2E), antibiotic treatment significantly decreased MHC-class II expression in spleen FO B2 cells of the WD group, as shown in Fig. 2H. Therefore, these data suggest that intestinal microbiota-induced atherosclerosis is characterized by B2-cell activation.

### Intestinal Microbiota Potentiates Activation of B2-cell TLR Signaling and Inflammatory Gene Expression during Atherogenesis

B cells exhibit variations in TLR expression patterns, which may directly transduce microbiota-derived signals. Alternatively, microbiota-derived signals via TLRs may modify B cell responses such as antibody production, antigen presentation, and cytokine secretion (Nickerson et al., 2010; Pasare and Medzhitov, 2005; Rawlings et al., 2012). The aforementioned results indicate that antibody responses are diminished via elimination of intestinal microbiota in WD-fed mice. Therefore, to further characterize these B2 cells and decipher whether B2-cell TLR signaling contributes to the microbiota-induced atherosclerosis observed in WD-fed mice, we isolated B2 cells from spleen and PVAT and quantified gene expression of TLR signaling. As expected, comparisons of the WD and WD-AT groups revealed a substantial difference in gene expression profiles (Fig. 3A and Tables 1, 2). Although TLR expression was unaffected (Fig. 5), the gene expression levels of proinflammatory cytokines were indeed elevated in FO B2 cells from PVAT of WD-fed mice. As the most striking difference, the transcription of the CD14 gene, which encodes surface antigen CD14, a molecule required for B-cell function, was significantly up-regulated in FO B cells of PVAT. Moreover, previous studies revealed that CD 14 is a component of the innate immune system and acts as a co-receptor along with TLR2 and TLR4 for the detection of bacterial lipopolysaccharide (LPS) (Heumann et al., 2001; Tasaka et al., 2003). In addition, the genes encoding inflammation-activated related molecules, including chemokine ligand 2 (Ccl2), heat shock 70 kDa protein 1a (Hspa1a), interleukin 1β (IL1β), interleukin 1 receptor, type 1 (IIl1r1), prostaglandin-endoperoxide synthase 2 (Ptgs2), and tumor necrosis factor receptor superfamily member 1a (Tnfrsf1a), were also up-regulated. In contrast to FO B2 cells from the spleen of WD-fed mice, the expressions of CCAAT/enhancer binding protein β (Cebpb) and NF-kappa-B inhibitor β (Nfkbib), which are negative regulators of TLR signaling, were induced in WD-AT mice, whereas the expressions of the inflammation-activated related genes Hspa1a and Ptgs2 were dramatically reduced (Table 1). These findings strongly suggest that the observed changes result from the elimination of the intestinal microbiota, and that the intestinal microbiota promotes atherosclerosis via activation of B2-cell TLR signaling pathway. B2-cell TLR signaling thus mediates microbiota-driven atherosclerosis. Taken together, these findings support a specific role of TLR signaling in B2 cells during microbiota-driven atherosclerosis.

**Table 1**

| Relative expression levels of the genes relevant to TLR signaling pathway in FO B cells (Fold Change) | | |
|---|---|---|
| | Relative fold changes in expression (*) | |
| Genes | FO B cells in spleen (WD-AT group) | FO B cells in PVAT (WD group) |
| Cc12 | -1.1481 | 17.8381 |
| Cd14 | 1.993 | 7.189 |
| Cebpb | 3.2331 | 1.1982 |
| Fos | 1.726 | -3.5108 |
| Hspa1a | -5.1166 | 9.3761 |
| Il1b | -1.0307 | 3.3433 |
| Il1r1 | 1.2616 | 3.5637 |
| Jun | 1.2743 | -2.16 |
| Nfkbib | 2.8025 | 1.5068 |
| Ptgs2 | -2.3499 | 3.9493 |
| Tnfrsf1a | 1.5827 | 2.7597 |

| | | |
|---|---|---|
| (*) The expression levels were relatively quantified by taking each gene expression level in spleen FO B cells of the WD group as a reference. | | |

**Table 2**

| Up-down regulation in the expression of genes relevant to TLR signaling pathway in MZ B cells in WD group vs. WD+AT group | |
|---|---|
| Genes | Relative fold changes in expression in MZ B cells (*) |
| Chuk | 3.8971 |
| Fos | 2.6863 |
| Hspa1a | -2.3842 |
| Ikbkb | 2.6361 |
| Irf1 | 2.0659 |
| Mapk8 | 2.6917 |
| Mapk8ip3 | 6.4522 |
| Mapk9 | 3.1227 |
| Tlr5 | -2.4133 |
| Tollip | 2.1339 |
| Tradd | 3.121 |
| Traf6 | 3.3725 |

| | |
|---|---|
| (*) The expression levels were relatively quantified by taking each gene expression level in MZ B cells of the WD group as a reference. | |

### B-cell Deficiency Attenuates Microbiota-induced Atherosclerosis

Because intestinal microbiota depletion may influence the development of atherosclerosis by reducing the number of activated B2 cells, we investigated whether B2-cell deficiency might afford protection against microbiota-induced atherosclerosis. A cohort of WD-fed mice was pretreated with a B-cell depleting agent, anti-mouse CD23 antibody (Purified Rat Anti-Mouse CD23, BD Pharmingen, B3B4, Isotype IgG2a). Intraperitoneal injections of anti-CD23 antibody were started one week before the development of atherosclerosis. Mice in the control group received saline. As expected, mice that received the anti-CD23 antibody had far fewer B2 cells in their spleens and PVAT than mice that received saline (Fig. 4A-4B). There were no changes in cell populations other than B2 cells (Fig. 8). Furthermore, it was found that WD-fed mice treated with anti-CD23 antibody gained weight in association with increased visceral and subcutaneous fat and serum lipid levels, similar to the WD-fed control mice (Fig. 4C-4I). However, after 8-week WD feeding, we compared plaques in WD-fed mice versus WD-fed plus anti-CD23 antibody-treated mice to find that WD-fed plus anti-CD23 antibody-treated mice exhibited a marked reduction in plaque formation (Fig. 4J-4K). Serum IgG and IgG3 levels were found to be elevated only in WD-fed mice untreated with the antibody (Fig. 4L-4M). These results confirmed that triggering of atherosclerosis by microbiota requires initial help from B2 cells. Altogether, these data indicate that microbiota aggravates atherosclerosis by stimulating activated B2-cell production and shifting the host response toward TH1-associated immunity.

### References

Ait-Oufella, H., Herbin, O., Bouaziz, J.D., Binder, C.J., Uyttenhove, C., Laurans, L., Taleb, S., Van Vre, E., Esposito, B., Vilar, J., et al. (2010). B cell depletion reduces the development of atherosclerosis in mice. J Exp Med 207, 1579-1587.
Balazs, M., Martin, F., Zhou, T., and Kearney, J.F. (2002). Blood Dendritic Cells Interact with Splenic Marginal Zone B Cells to Initiate T-Independent Immune Responses. Immunity 17, 341-352.
Barthel, M., Hapfelmeier, S., Quintanilla-Martinez, L., Kremer, M., Rohde, M., Hogardt, M., Pfeffer, K., Russmann, H., and Hardt, W.-D. (2003). Pretreatment of Mice with Streptomycin Provides a Salmonella enterica Serovar Typhimurium Colitis Model That Allows Analysis of Both Pathogen and Host. Infection and Immunity 71, 2839-2858.
Brown, J.M., and Hazen, S.L. (2015). The gut microbial endocrine organ: bacterially derived signals driving cardiometabolic diseases. Annu Rev Med 66, 343-359.
Canducci, F., Saita, D., Foglieni, C., Piscopiello, M.R., Chiesa, R., Colombo, A., Cianflone, D., Maseri, A., Clementi, M., and Burioni, R. (2012). Cross-reacting antibacterial auto-antibodies are produced within coronary atherosclerotic plaques of acute coronary syndrome patients. PLoS One 7, e42283.
Cerutti, A., Puga, I., and Cols, M. (2012). New helping friends for B cells. Eur J Immunol 42, 1956-1968.
Coyle, A.J., Wagner, K., Bertrand, C., Tsuyuki, S., Bews, J., and Heusser, C. (1996). Central role of immunoglobulin (Ig) E in the induction of lung eosinophil infiltration and T helper 2 cell cytokine production: inhibition by a non-anaphylactogenic anti-IgE antibody. The Journal of Experimental Medicine 183, 1303-1310.
Croswell, A., Amir, E., Teggatz, P., Barman, M., and Salzman, N.H. (2009). Prolonged impact of antibiotics on intestinal microbial ecology and susceptibility to enteric Salmonella infection. Infect Immun 77, 2741-2753.
Dasic, G., Juillard, P., Graber, P., Herren, S., Angell, T., Knowles, R., Bonnefoy, J.-Y., Kosco-Vilbois, M.H., and Chvatchko, Y. (1999). Critical role of CD23 in allergen-induced bronchoconstriction in a murine model of allergic asthma. European Journal of Immunology 29, 2957-2967.
Doran, A.C., Lipinski, M.J., Oldham, S.N., Garmey, J.C., Campbell, K.A., Skaflen, M.D., Cutchins, A., Lee, D.J., Glover, D.K., Kelly, K.A., et al. (2012). B-cell aortic homing and atheroprotection depend on Id3. Circ Res 110, e1-12.
Drosos, I., Tavridou, A., and Kolios, G. (2015). New aspects on the metabolic role of intestinal microbiota in the development of atherosclerosis. Metabolism 64, 476-481.
Edfeldt, K., Swedenborg, J., Hansson, G.K., and Yan, Z.-q. (2002). Expression of Toll-Like Receptors in Human Atherosclerotic Lesions. A Possible Pathway for Plaque Activation 105, 1158-1161.
Emtiazy, M., Choopani, R., Khodadoost, M., Tansaz, M., and Nazem, E. (2013). Atheroprotector role of the spleen based on the teaching of Avicenna (Ibn Sina). Int J Cardiol 167, 26-28.
Fu, J., Bonder, M.J., Cenit, M.C., Tigchelaar, E.F., Maatman, A., Dekens, J.A., Brandsma, E., Marczynska, J., Imhann, F., Weersma, R.K., et al. (2015). The Gut Microbiome Contributes to a Substantial Proportion of the Variation in Blood Lipids. Circ Res 117, 817-824.
Grabner, R., Lotzer, K., Dopping, S., Hildner, M., Radke, D., Beer, M., Spanbroek, R., Lippert, B., Reardon, C.A., Getz, G.S., et al. (2009). Lymphotoxin β receptor signaling promotes tertiary lymphoid organogenesis in the aorta adventitia of aged ApoE-/- mice. The Journal of Experimental Medicine 206, 233-248.
Guinamard, R., Okigaki, M., Schlessinger, J., and Ravetch, J.V. (2000). Absence of marginal zone B cells in Pyk-2-deficient mice defines their role in the humoral response. Nat Immunol 1, 31-36.
Hamze, M., Desmetz, C., Berthe, M.L., Roger, P., Boulle, N., Brancherau, P., Picard, E., Guzman, C., Tolza, C., and Guglielmi, P. (2013). Characterization of resident B cells of vascular walls in human atherosclerotic patients. J Immunol 191, 3006-3016.
Hashimoto, K., Cohen, R.N., Yamada, M., Markan, K.R., Monden, T., Satoh, T., Mori, M., and Wondisford, F.E. (2006). Cross-talk between thyroid hormone receptor and liver X receptor regulatory pathways is revealed in a thyroid hormone resistance mouse model. J Biol Chem 281, 295-302.
Hermansson, A., Ketelhuth, D.F., Strodthoff, D., Wurm, M., Hansson, E.M., Nicoletti, A., Paulsson-Berne, G., and Hansson, G.K. (2010). Inhibition of T cell response to native low-density lipoprotein reduces atherosclerosis. J Exp Med 207, 1081-1093.
Heumann, D., Adachi, Y., Le Roy, D., Ohno, N., Yadomae, T., Glauser, M.P., and Calandra, T. (2001). Role of plasma, lipopolysaccharide-binding protein, and CD14 in response of mouse peritoneal exudate macrophages to endotoxin. Infect Immun 69, 378-385.
Hilgendorf, I., Theurl, I., Gerhardt, L.M., Robbins, C.S., Weber, G.F., Gonen, A., Iwamoto, Y., Degousee, N., Holderried, T.A., Winter, C., et al. (2014). Innate response activator B cells aggravate atherosclerosis by stimulating T helper-1 adaptive immunity. Circulation 129, 1677-1687.
Hosoi, T., Yokoyama, S., Matsuo, S., Akira, S., and Ozawa, K. (2010). Myeloid differentiation factor 88 (MyD88)-deficiency increases risk of diabetes in mice. PLoS One 5.
Irie, D., Kawahito, H., Wakana, N., Kato, T., Kishida, S., Kikai, M., Ogata, T., Ikeda, K., Ueyama, T., Matoba, S., et al. (2015). Transplantation of periaortic adipose tissue from angiotensin receptor blocker-treated mice markedly ameliorates atherosclerosis development in apoE-/- mice. J Renin Angiotensin Aldosterone Syst 16, 67-78.
Ishigami, T., Abe, K., Aoki, I., Minegishi, S., Ryo, A., Matsunaga, S., Matsuoka, K., Takeda, H., Sawasaki, T., Umemura, S., et al. (2013). Anti-interleukin-5 and multiple autoantibodies are associated with human atherosclerotic diseases and serum interleukin-5 levels. FASEB J 27, 3437-3445.
Jiang, Y., Arase, N., Kohyama, M., Hirayasu, K., Suenaga, T., Jin, H., Matsumoto, M., Shida, K., Lanier, L.L., Saito, T., et al. (2013). Transport of misfolded endoplasmic reticulum proteins to the cell surface by MHC class II molecules. International Immunology 25, 235-246.
Jin, H., Arase, N., Hirayasu, K., Kohyama, M., Suenaga, T., Saito, F., Tanimura, K., Matsuoka, S., Ebina, K., Shi, K., et al. (2014). Autoantibodies to IgG/HLA class II complexes are associated with rheumatoid arthritis susceptibility. Proc Natl Acad Sci U S A 111, 3787-3792.
Kawai, T., and Akira, S. (2010). The role of pattern-recognition receptors in innate immunity: update on Toll-like receptors. Nat Immunol 11, 373-384.
Koeth, R.A., Wang, Z., Levison, B.S., Buffa, J.A., Org, E., Sheehy, B.T., Britt, E.B., Fu, X., Wu, Y., Li, L., et al. (2013). Intestinal microbiota metabolism of L-carnitine, a nutrient in red meat, promotes atherosclerosis. Nat Med 19, 576-585.
Koren, O., Spor, A., Felin, J., Fak, F., Stombaugh, J., Tremaroli, V., Behre, C.J., Knight, R., Fagerberg, B., Ley, R.E., et al. (2011). Human oral, gut, and plaque microbiota in patients with atherosclerosis. Proceedings of the National Academy of Sciences 108, 4592-4598.
Kyaw, T., Tay, C., Khan, A., Dumouchel, V., Cao, A., To, K., Kehry, M., Dunn, R., Agrotis, A., Tipping, P., et al. (2010). Conventional B2 B Cell Depletion Ameliorates whereas Its Adoptive Transfer Aggravates Atherosclerosis. The Journal of Immunology 185, 4410-4419.
Kyaw, 1., Tay, C., Krishnamurthi, S., Kanellakis, P., Agrotis, A., Tipping, P., Bobik, A., and Toh, B.-H. (2011). B1a B Lymphocytes Are Atheroprotective by Secreting Natural IgM That Increases IgM Deposits and Reduces Necrotic Cores in Atherosclerotic Lesions. Circulation Research 109, 830-840.
Lammers, B., Zhao, Y., Foks, A.C., Hildebrand, R.B., Kuiper, J., Van Berkel, T.J., and Van Eck, M. (2012). Leukocyte ABCA1 remains atheroprotective in splenectomized LDL receptor knockout mice. PLoS One 7, e48080.
Le Chatelier, E., Nielsen, T., Qin, J., Prifti, E., Hildebrand, F., Falony, G., Almeida, M., Arumugam, M., Batto, J.-M., Kennedy, S., et al. (2013). Richness of human gut microbiome correlates with metabolic markers. Nature 500, 541-546.
Lecoanet-Henchoz, S., Gauchat, J.-F., Aubry, J.-P., Graber, P., Life, P., Paul-Eugene, N., Ferrua, B., Corbi, A.L., Dugas, B., Plater-Zyberk, C., et al. (1995). CD23 Regulates monocyte activation through a novel interaction with the adhesion molecules CD11b-CD18 and CD11c-CD18. Immunity 3, 119-125.
LeibundGut-Landmann, S., Waldburger, J.-M., e Sousa, C.R., Acha-Orbea, H., and Reith, W. (2004). MHC class II expression is differentially regulated in plasmacytoid and conventional dendritic cells. Nat Immunol 5, 899-908.
Libby, P. (2002). Inflammation in atherosclerosis. Nature 420, 868-874.
Lipinski, M.J., Perry, H.M., Doran, A.C., Oldham, S.N., and McNamara, C.A. (2011). Comment on "Conventional B2 B Cell Depletion Ameliorates whereas Its Adoptive Transfer Aggravates Atherosclerosis". The Journal of Immunology 186, 4.
Martin, F., and Kearney, J.F. (2002). Marginal-zone B cells. Nat Rev Immunol 2, 323-335.
Nickerson, K.M., Christensen, S.R., Shupe, J., Kashgarian, M., Kim, D., Elkon, K., and Shlomchik, M.J. (2010). TLR9 Regulates TLR7- and MyD88-Dependent Autoantibody Production and Disease in a Murine Model of Lupus. The Journal of Immunology 184, 1840-1848.
Nicoletti, A., Kaveri, S., Caligiuri, G., Bari, xE, ty, J., and Hansson, G.K. (1998). Immunoglobulin treatment reduces atherosclerosis in apo E knockout mice. The Journal of Clinical Investigation 102, 910-918.
Panda, S., Zhang, J., Tan, N.S., Ho, B., and Ding, J.L. (2013). Natural IgG antibodies provide innate protection against ficolin-opsonized bacteria. The EMBO Journal 32, 2905-2919.
Pasare, C., and Medzhitov, R. (2005). Control of B-cell responses by Toll-like receptors. Nature 438, 364-368.
Puga, I., Cols, M., Barra, C.M., He, B., Cassis, L., Gentile, M., Comerma, L., Chorny, A., Shan, M., Xu, W., et al. (2012). B cell-helper neutrophils stimulate the diversification and production of immunoglobulin in the marginal zone of the spleen. Nat Immunol 13, 170-180.
Rakoff-Nahoum, S., Paglino, J., Eslami-Varzaneh, F., Edberg, S., and Medzhitov, R. (2004). Recognition of commensal microflora by toll-like receptors is required for intestinal homeostasis. Cell 118, 229-241.
Rawlings, D.J., Schwartz, M.A., Jackson, S.W., and Meyer-Bahlburg, A. (2012). Integration of B cell responses through Toll-like receptors and antigen receptors. Nat Rev Immunol 12, 282-294.
Rezende, A.B., Neto, N.N., Fernandes, L.R., Ribeiro, A.C., Alvarez-Leite, J.I., and Teixeira, H.C. (2011). Splenectomy increases atherosclerotic lesions in apolipoprotein E deficient mice. J Surg Res 171, e231-236.
Serino, M., Blasco-Baque, V., Nicolas, S., and Burcelin, R. (2014). Far from the eyes, close to the heart: dysbiosis of gut microbiota and cardiovascular consequences. Curr Cardiol Rep 16, 540.
Shapiro-Shelef, M., and Calame, K. (2005). Regulation of plasma-cell development. Nat Rev Immunol 5, 230-242.
Spence, J.D., Urquhart, B.L., and Bang, H. (2016). Effect of renal impairment on atherosclerosis: only partially mediated by homocysteine. Nephrol Dial Transplant 31, 937-944.
Stief, A., Texido, G., Sansig, G., Eibel, H., Le Gros, G., and van der Putten, H. (1994). Mice deficient in CD23 reveal its modulatory role in IgE production but no role in T and B cell development. The Journal of Immunology 152, 3378-3390.
Sun, K., Kusminski, C.M., and Scherer, P.E. (2011). Adipose tissue remodeling and obesity. The Journal of Clinical Investigation 121, 2094-2101.
Tang, W.H., and Hazen, S.L. (2014). The contributory role of gut microbiota in cardiovascular disease. J Clin Invest 124, 4204-4211.
Tang, W.H., Wang, Z., Levison, B.S., Koeth, R.A., Britt, E.B., Fu, X., Wu, Y., and Hazen, S.L. (2013). Intestinal microbial metabolism of phosphatidylcholine and cardiovascular risk. N Engl J Med 368, 1575-1584.
Tasaka, S., Ishizaka, A., Yamada, W., Shimizu, M., Koh, H., Hasegawa, N., Adachi, Y., and Yamaguchi, K. (2003). Effect of CD14 blockade on endotoxin-induced acute lung injury in mice. Am J Respir Cell Mol Biol 29, 252-258.
Tsiantoulas, D., Diehl, C.J., Witztum, J.L., and Binder, C.J. (2014). B cells and humoral immunity in atherosclerosis. Circ Res 114, 1743-1756.
Velagapudi, V.R., Hezaveh, R., Reigstad, C.S., Gopalacharyulu, P., Yetukuri, L., Islam, S., Felin, J., Perkins, R., Boren, J., Oresic, M., et al. (2010). The gut microbiota modulates host energy and lipid metabolism in mice. J Lipid Res 51, 1101-1112.
Victora, G.D. (2014). ILCs in the zone. Nat Immunol 15, 313-314.
Vinuesa, C.G., and Chang, P.P. (2013). Innate B cell helpers reveal novel types of antibody responses. Nat Immunol 14, 119-126.
Wang, Z., Klipfell, E., Bennett, B.J., Koeth, R., Levison, B.S., Dugar, B., Feldstein, A.E., Britt, E.B., Fu, X., Chung, Y.M., et al. (2011). Gut flora metabolism of phosphatidylcholine promotes cardiovascular disease. Nature 472, 57-63.
Wardemann, H., Boehm, T., Dear, N., and Carsetti, R. (2002). B-1a B Cells that Link the Innate and Adaptive Immune Responses Are Lacking in the Absence of the Spleen. The Journal of Experimental Medicine 195, 771-780.
Weill, J.-C., Weller, S., and Reynaud, C.-A. (2009). Human Marginal Zone B Cells. Annual Review of Immunology 27, 267-285.
Wick, G., Knoflach, M., and Xu, Q. (2004). Autoimmune and inflammatory mechanisms in atherosclerosis. Annu Rev Immunol 22, 361-403.

## Claims

1. An antiarteriosclerotic agent comprising, as an active ingredient, a substance which selectively suppresses B2 cells.

2. The antiarteriosclerotic agent according to claim 1, wherein the substance which selectively suppresses B2 cells is an anti-CD23 antibody.

3. A method of assisting in determining a condition of arteriosclerosis, comprising measuring expression, using a sample isolated from a subject, of at least one gene selected from the group consisting of the following gene group A and gene group B:
gene group A: Ccl2, Cdl4, Hspa1a, Il1b, Il1r1, Ptgs2, Tnfrsf1a, Tlr5;
gene group B: Cebpb, Fos, Jun, Nfkbib, Chuk, Ikbkb, Irf1, Mapk8, Mapk8ip3, Mapk9, Tollip, Tradd.

4. The method according to claim 3, wherein the subject is a patient with arteriosclerosis.

5. The method according to claim 3 or 4, wherein the sample comprises B2 cells.

6. The method according to any one of claims 3 to 5, wherein the genes in the gene group A are the genes whose high expression indicates presence or progression of arteriosclerosis, and the genes in the gene group B are the genes whose low expression indicates presence or progression of arteriosclerosis.

7. A method of assisting in determining a condition of arteriosclerosis, comprising measuring the level of IgG3 and/or the level of total IgG in a serum sample isolated from a subject.
